# EUROPEAN PATENT SPECIFICATION

(11) **EP 3 096 772 B1**
(45) Date of publication and mention of the grant of the patent: **26.12.2018**
(21) Application number: 15703742.5
(22) Date of filing: 22.01.2015
(51) Int. Cl.: A61K 38/17, A61K 39/00, A61K 31/47, A61K 45/00, A61K 38/22, A61K 31/4709

(54) **METHODS AND PHARMACEUTICAL COMPOSITIONS FOR THE TREATMENT OF HEPATOCELLULAR CARCINOMAS**
VERFAHREN UND PHARMAZEUTISCHE ZUSAMMENSETZUNGEN ZUR BEHANDLUNG VON HEPATOZELLULÄREN KARZINOMEN
PROCÉDÉS ET COMPOSITIONS PHARMACEUTIQUES POUR LE TRAITEMENT DE CARCINOMES HÉPATOCELLULAIRES

(30) Priority: 22.01.2014 EP 14305085
(43) Date of publication of application: 30.11.2016
(73) Proprietor: INSERM (Institut National de la Santé et de la Recherche Médicale), 75013 Paris (FR); Assistance Publique-Hôpitaux de Paris (APHP), 75004 Paris (FR); Université de Versailles Saint-Quentin-en-Yvelines, 78000 Versailles (FR); Université Paris Diderot - Paris 7, 75013 Paris (FR)
(72) Inventor: COUVINEAU, Alain, F-75870 Paris Cedex 18 (FR); VOISIN, Thierry, F-75870 Paris Cedex 18 (FR); COUVELARD, Anne, 16 Rue Henri Huchard F-75018 Paris (FR)
(74) Representative: Inserm Transfert
(86) International application number: PCT/EP2015/051279
(87) International publication number: WO 2015/110547

(56) References cited:
- WO-A1-2011/057471
- T VOISIN ET AL.: "Aberrant expression of OX1 receptors for orexin in colon cancers and liver metastases: an openable gate to apoptosis", CANCER RESEARCH, vol. 71, 17 March 2011 (2011-03-17), pages 3341-33 51, XP055124207, American Association for Cancer Research ISSN: 0008-5472 cited in the application
- PATRICIA ROUET-BENZINEB ET AL.: "Orexins acting at native OX1, receptor in colon cancer and neuroblastoma cells or at recombinant OX1 receptor suppress cell growth by inducing apoptosis", JOURNAL OF BIOLOGICAL CHEMISTRY, vol. 279, no. 44, 29 October 2004 (2004-10-29), pages 45875-45886, XP55124216, USA

### SEQUENCE LISTING

<110> Inserm
<120> METHODS AND PHARMACEUTICAL COMPOSITIONS FOR THE TREATMENT OF HEPATOCELLULAR CARCINOMAS
<130> BI013264 COUVINEAU / MC
<160> 3
<170> PatentIn version 3.3
<210> 1
   <211> 425
   <212> PRT
   <213> Homo sapiens
<400> 1
   Met Glu Pro Ser Ala Thr Pro Gly Ala Gln Met Gly Val Pro Pro Gly
   1               5                   10                  15
   Ser Arg Glu Pro Ser Pro Val Pro Pro Asp Tyr Glu Asp Glu Phe Leu
               20                  25                  30
   Arg Tyr Leu Trp Arg Asp Tyr Leu Tyr Pro Lys Gln Tyr Glu Trp Val
           35                  40                  45
   Leu Ile Ala Ala Tyr Val Ala Val Phe Val Val Ala Leu Val Gly Asn
       50                  55                  60
   Thr Leu Val Cys Leu Ala Val Trp Arg Asn His His Met Arg Thr Val
   65                  70                  75                  80
   Thr Asn Tyr Phe Ile Val Asn Leu Ser Leu Ala Asp Val Leu Val Thr
                   85                  90                  95
   Ala Ile Cys Leu Pro Ala Ser Leu Leu Val Asp Ile Thr Glu Ser Trp
               100                 105                 110
   Leu Phe Gly His Ala Leu Cys Lys Val Ile Pro Tyr Leu Gln Ala Val
           115                 120                 125
   Ser Val Ser Val Ala Val Leu Thr Leu Ser Phe Ile Ala Leu Asp Arg
       130                 135                 140
   Trp Tyr Ala Ile Cys His Pro Leu Leu Phe Lys Ser Thr Ala Arg Arg
   145                 150                 155                 160
   Ala Arg Gly Ser Ile Leu Gly Ile Trp Ala Val Ser Leu Ala Ile Met
                   165                 170                 175
   Val Pro Gln Ala Ala Val Met Glu Cys Ser Ser Val Leu Pro Glu Leu
               180                 185                 190
   Ala Asn Arg Thr Arg Leu Phe Ser Val Cys Asp Glu Arg Trp Ala Asp
           195                 200                 205
   Asp Leu Tyr Pro Lys Ile Tyr His Ser Cys Phe Phe Ile Val Thr Tyr
       210                 215                 220
   Leu Ala Pro Leu Gly Leu Met Ala Met Ala Tyr Phe Gln Ile Phe Arg
   225                 230                 235                 240
   Lys Leu Trp Gly Arg Gln Ile Pro Gly Thr Thr Ser Ala Leu Val Arg
                   245                 250                 255
   Asn Trp Lys Arg Pro Ser Asp Gln Leu Gly Asp Leu Glu Gln Gly Leu
               260                 265                 270
   Ser Gly Glu Pro Gln Pro Arg Gly Arg Ala Phe Leu Ala Glu Val Lys
           275                 280                 285
   Gln Met Arg Ala Arg Arg Lys Thr Ala Lys Met Leu Met Val Val Leu
       290                 295                 300
   Leu Val Phe Ala Leu Cys Tyr Leu Pro Ile Ser Val Leu Asn Val Leu
   305                 310                 315                 320
   Lys Arg Val Phe Gly Met Phe Arg Gln Ala Ser Asp Arg Glu Ala Val
                   325                 330                 335
   Tyr Ala Cys Phe Thr Phe Ser His Trp Leu Val Tyr Ala Asn Ser Ala
               340                 345                 350
   Ala Asn Pro Ile Ile Tyr Asn Phe Leu Ser Gly Lys Phe Arg Glu Gln
           355                 360                 365
   Phe Lys Ala Ala Phe Ser Cys Cys Leu Pro Gly Leu Gly Pro Cys Gly
       370                 375                 380
   Ser Leu Lys Ala Pro Ser Pro Arg Ser Ser Ala Ser His Lys Ser Leu
   385                 390                 395                 400
   Ser Leu Gln Ser Arg Cys Ser Ile Ser Lys Ile Ser Glu His Val Val
                   405                 410                 415
   Leu Thr Ser Val Thr Thr Val Leu Pro
               420                 425
<210> 2
   <211> 33
   <212> PRT
   <213> Homo sapiens
<220>
   <221> MISC_FEATURE
   <222> (1)..(1)
   <223> X is pyruvoglutamate
<220>
   <221> MISC_FEATURE
   <222> (1)..(1)
   <223> X is pyroglutamate
<400> 2
   Xaa Pro Leu Pro Asp Cys Cys Arg Gln Lys Thr Cys Ser Cys Arg Leu
   1               5                   10                  15
   Tyr Glu Leu Leu His Gly Ala Gly Asn His Ala Ala Gly Ile Leu Thr
               20                  25                  30
   Leu
<210> 3
   <211> 27
   <212> PRT
   <213> Homo sapiens
<400> 3
   Arg Gly Pro Pro Gly Leu Gln Gly Arg Leu Gln Arg Leu Leu Gln Ala
   1               5                   10                  15
   Ser Gly Asn His Ala Ala Gly Ile Leu Thr Met
               20                  25

## Description

### FIELD OF THE INVENTION:

The present invention relates to methods and pharmaceutical compositions for the treatment of hepatocellular carcinomas.

### BACKGROUND OF THE INVENTION:

Hepatocellular carcinoma (HCC) is the fifth most common cancer worldwide and the third most common cause of cancer-related deaths. The disease is often diagnosed late in the course of clinical manifestation. As a result, only 10-15% of patients are candidates for curative surgery. For the majority of HCC patients, systemic chemotherapies or supportive therapies are the mainstay treatment options. Nevertheless, most chemotherapeutic agents show limited effectiveness and have not been able to improve patient survival. The adverse clinical course of most HCC patients underscores much need for more efficacious chemotherapies and development of targeting strategies.

The orexins (hypocretins) comprise two neuropeptides produced in the hypothalamus: the orexin A (OX-A) (a 33 amino acid peptide) and the orexin B (OX-B) (a 28 amino acid peptide) (Sakurai T. et al., Cell, 1998, 92, 573-585). Orexins are found to stimulate food consumption in rats suggesting a physiological role for these peptides as mediators in the central feedback mechanism that regulates feeding behaviour. Orexins regulate states of sleep and wakefulness opening potentially novel therapeutic approaches for narcoleptic or insomniac patients. Orexins have also been indicated as playing a role in arousal, reward, learning and memory. Two orexin receptors have been cloned and characterized in mammals. They belong to the super family of G-protein coupled receptors (7-transmembrane spanning receptor) (Sakurai T. et al., Cell, 1998, 92, 573-585): the orexin-1 receptor (OX1R or HCTR1) is more selective for OX-A than OX-B and the orexin-2 receptor (OX2R or HCTR2) binds OX-A as well as OX-B. A recent study shows that activation of OX1R by orexin can promote robust in vitro and in vivo apoptosis in colon cancer cells even when they are resistant to the most commonly used drug in colon cancer chemotherapy (Voisin T, El Firar A, Fasseu M, Rouyer-Fessard C, Descatoire V, Walker F, Paradis V, Bedossa P, Henin D, Lehy T, Laburthe M. Aberrant expression of OX1 receptors for orexins in colon cancers and liver metastases: an openable gate to apoptosis. Cancer Res. 2011 May 1;71(9):3341-51). Remarkably, all primary colorectal tumors regardless of their localization and Duke's stages expressed OX1R while adjacent normal colonocytes as well as control normal tissues were negative. Thus this study supports that OX1R is an Achilles's heel of colon cancers (even chemoresistance) and suggests that OX1R agonists might be novel candidates for colon cancer therapy.

### SUMMARY OF THE INVENTION:

The present invention relates to methods and pharmaceutical compositions for the treatment of hepatocellular carcinomas. In particular, the present invention is defined by the claims.

### DETAILED DESCRIPTION OF THE INVENTION:

The present invention relates to an OX1R agonist for use in the treatment of hepatocellular carcinoma in a subject in need thereof.

As used herein, the tem "OX1R" has its general meaning in the art and refers to the 7-transmembrane spanning receptor OX1R for orexins. According to the invention, OX1R promotes apoptosis in the human prancreatic cancer cell line through a mechanism which is not related to Gq-mediated phopholipase C activation and cellular calcium transients. Orexins induce indeed tyrosine phosphorylation of 2 tyrosine-based motifs in OX1R, ITIM and ITSM, resulting in the recruitment of the phosphotyrosine phosphatase SHP-2, the activation of which is responsible for mitochondrial apoptosis (Voisin T, El Firar A, Rouyer-Fessard C, Gratio V, Laburthe M. A hallmark of immunoreceptor, the tyrosine-based inhibitory motif ITIM, is present in the G protein-coupled receptor OX1R for orexins and drives apoptosis: a novel mechanism. FASEB J. 2008 Jun;22(6):1993-2002.;El Firar A, Voisin T, Rouyer-Fessard C, Ostuni MA, Couvineau A, Laburthe M. Discovery of a functional immunoreceptor tyrosine-based switch motif in a 7-transmembrane-spanning receptor: role in the orexin receptor OXIR-driven apoptosis. FASEB J. 2009 Dec;23(12):4069-80. doi: 10.1096/fj.09-131367. Epub 2009 Aug 6.). An exemplary amino acid sequence of OX1R is shown as SEQ ID NO:1.
orexin receptor-1 OX1R (SEQ ID NO:1)

Accordingly, as used herein, the term "OX1R agonist" refers to any compound that is able to bind to OX1R and promotes OX1R activity which consists of activation of signal transduction pathways involving recruitment of SHP-2 and the induction of apoptosis of the cell, independently of transient calcium release.

In some embodiment, the OX1R agonist is an OX1R antibody or a portion thereof.

As used herein, "antibody" includes both naturally occurring and non-naturally occurring antibodies. Specifically, "antibody" includes polyclonal and monoclonal antibodies, and monovalent and divalent fragments thereof. Furthermore, "antibody" includes chimeric antibodies, wholly synthetic antibodies, single chain antibodies, and fragments thereof. The antibody may be a human or nonhuman antibody. A nonhuman antibody may be humanized by recombinant methods to reduce its immunogenicity in man.

In some embodiments of the antibodies or portions thereof described herein, the antibody is a monoclonal antibody. In some embodiments of the antibodies or portions thereof described herein, the antibody is a polyclonal antibody. In some embodiments of the antibodies or portions thereof described herein, the antibody is a humanized antibody. In some embodiments of the antibodies or portions thereof described herein, the antibody is a chimeric antibody. In some embodiments of the antibodies or portions thereof described herein, the portion of the antibody comprises a light chain of the antibody. In some embodiments of the antibodies or portions thereof described herein, the portion of the antibody comprises a heavy chain of the antibody. In some embodiments of the antibodies or portions thereof described herein, the portion of the antibody comprises a Fab portion of the antibody. In some embodiments of the antibodies or portions thereof described herein, the portion of the antibody comprises a F(ab')2 portion of the antibody. In some embodiments of the antibodies or portions thereof described herein, the portion of the antibody comprises a Fc portion of the antibody. In some embodiments of the antibodies or portions thereof described herein, the portion of the antibody comprises a Fv portion of the antibody. In some embodiments of the antibodies or portions thereof described herein, the portion of the antibody comprises a variable domain of the antibody. In some embodiments of the antibodies or portions thereof described herein, the portion of the antibody comprises one or more CDR domains of the antibody.

Antibodies are prepared according to conventional methodology. Monoclonal antibodies may be generated using the method of Kohler and Milstein (Nature, 256:495, 1975). To prepare monoclonal antibodies useful in the invention, a mouse or other appropriate host animal is immunized at suitable intervals (e.g., twice-weekly, weekly, twice-monthly or monthly) with antigenic forms of OX1R. The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes. Briefly, the recombinant OX1R may be provided by expression with recombinant cell lines. In particular, OX1R may be provided in the form of human cells expressing OX1R at their surface. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

Significantly, as is well-known in the art, only a small portion of an antibody molecule, the paratope, is involved in the binding of the antibody to its epitope (see, in general, Clark, W. R. (1986) The Experimental Foundations of Modern Immunology Wiley & Sons, Inc., New York; Roitt, I. (1991) Essential Immunology, 7th Ed., Blackwell Scientific Publications, Oxford). The Fc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDRS). The CDRs, and in particular the CDRS regions, and more particularly the heavy chain CDRS, are largely responsible for antibody specificity.

It is now well-established in the art that the non CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody.

This invention provides in certain embodiments compositions and methods that include humanized forms of antibodies. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567,5,225,539,5,585,089, 5,693,761, 5,693,762 and 5,859,205,. The above U.S. Pat. Nos. 5,585,089 and 5,693,761, and WO 90/07861 also propose four possible criteria which may used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3A of the CDRs in a three dimensional model of the antibody and is predicted to be capable of interacting with the CDRs. The above methods are merely illustrative of some of the methods that one skilled in the art could employ to make humanized antibodies. One of ordinary skill in the art will be familiar with other methods for antibody humanization.

In some embodiments of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgG1, IgG2, IgG3, IgG4, IgA and IgM molecules. A "humanized" antibody retains a similar antigenic specificity as the original antibody. However, using certain methods of humanization, the affinity and/or specificity of binding of the antibody may be increased using methods of "directed evolution", as described by Wu et al., /. Mol. Biol. 294:151, 1999.

Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein, These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans.

In vitro methods also exist for producing human antibodies. These include phage display technology (U.S. Pat. Nos. 5,565,332 and 5,573,905) and in vitro stimulation of human B cells (U.S. Pat. Nos. 5,229,275 and 5,567,610).

Thus, as will be apparent to one of ordinary skill in the art, the present invention also provides for F(ab') 2 Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

The various antibody molecules and fragments may derive from any of the commonly known immunoglobulin classes, including but not limited to IgA, secretory IgA, IgE, IgG and IgM. IgG subclasses are also well known to those in the art and include but are not limited to human IgG1, IgG2, IgG3 and IgG4.

In another embodiment, the antibody according to the invention is a single domain antibody. The term "single domain antibody" (sdAb) or "VHH" refers to the single heavy chain variable domain of antibodies of the type that can be found in Camelid mammals which are naturally devoid of light chains. Such VHH are also called "nanobody®". According to the invention, sdAb can particularly be llama sdAb.

In some embodiments of the agents described herein, the agent is a polypeptide. In a particular embodiment the polypeptide is a functional equivalent of Orexin-A or Orexin-B.

As used herein the term "orexin-A" has its general meaning in the art and refers to the amino acid sequence as shown by SEQ ID NO:2.

Orexin-A (SEQ ID NO:2): ₚeplpdccrqk tcscrlyell hgagnhaagi ltl wherein ₚe means pyroglutamate.

As used herein the term "orexin-B" has its general meaning in the art and refers to the amino acid sequence as shown by SEQ ID NO:3.

Orexin-B (SEQ ID NO:3): rsgppglqgr lqrllqasgn haagiltm

As used herein, also included is a polypeptide which is capable of binding to OX1R, thereby promoting an OX1R activity according to the invention. The term "functional equivalent" includes fragments, mutants, and muteins of Orexin-A and Orexin-B. The term "functionally equivalent" thus includes any equivalent of orexins (i.e. Orexin-A or Orexin-B) obtained by altering the amino acid sequence, for example by one or more amino acid deletions, substitutions or additions such that the protein analogue retains the ability to bind to OX1R and promote an OX1R activity according to the invention (e.g. aoptosis of the cancer cell). Amino acid substitutions may be made, for example, by point mutation of the DNA encoding the amino acid sequence.

In some embodiments, the functional equivalent is at least 80% homologous (i.e. 80, 81, 82, 83, 84, 85, 86, 87, 88, 89, 90, 91, 92, 93, 94, 95, 96, 97, 98, 99, or 100% homologous) to the corresponding protein. In some embodiments, the functional equivalent is at least 90% homologous (i.e. 90, 91, 92, 93, 94, 95, 96, 97, 98, 99, or 100% homologous) as assessed by any conventional analysis algorithm such as for example, the Pileup sequence analysis software (Program Manual for the Wisconsin Package, 1996). The term "a functionally equivalent fragment" as used herein also may mean any fragment or assembly of fragments of Orexin that binds to OX1R and promote the OX1R activity according to the invention. Accordingly the present invention provides a polypeptide which comprises consecutive amino acids having a sequence which corresponds to the sequence of at least a portion of Orexin-A or Orexin-B, which portion binds to OX1R and promotes the OX1R activity according to the invention. Functionally equivalent fragments may belong to the same protein family as the human Orexins identified herein. By "protein family" is meant a group of proteins that share a common function and exhibit common sequence homology. Homologous proteins may be derived from non-human species. In particular, the homology between functionally equivalent protein sequences is at least 80% across the whole of the sequence. More In particular, homology is greater than 90% across the whole of the sequence. More In particular, homology is greater than 95% across the whole of the sequence.

In some embodiments, the last residue of SEQ ID NO:3, i.e. the methionine residue at position 28 is amidated. As used herein, the term "amidation," has its general meaning in the art and refers to the process consisting of producing an amide moiety.

The polypeptides of the invention may be produced by any suitable means, as will be apparent to those of skill in the art. In order to produce sufficient amounts of polypeptides or functional equivalents thereof for use in accordance with the present invention, expression may conveniently be achieved by culturing under appropriate conditions recombinant host cells containing the polypeptide of the invention. In particular, the polypeptide is produced by recombinant means, by expression from an encoding nucleic acid molecule. Systems for cloning and expression of a polypeptide in a variety of different host cells are well known. When expressed in recombinant form, the polypeptide is in particular generated by expression from an encoding nucleic acid in a host cell. Any host cell may be used, depending upon the individual requirements of a particular system. Suitable host cells include bacteria mammalian cells, plant cells, yeast and baculovirus systems. Mammalian cell lines available in the art for expression of a heterologous polypeptide include Chinese hamster ovary cells. HeLa cells, baby hamster kidney cells and many others. Bacteria are also preferred hosts for the production of recombinant protein, due to the ease with which bacteria may be manipulated and grown. A common, preferred bacterial host is *E coli.*

Methods for producing amidated polypeptide are well known in the art and typically involve use of amidation enzyme. As used herein, the term "amidation enzyme" is defined as the enzymes which can convert the carboxyl group of a polypeptide to an amide group. Enzymes capable of C-terminal amidation of peptides have been known for a long time (Eipper et al. Mol. Endocrinol. 1987 November; 1 (11): 777). Examples of amidating enzymes include peptidylglycine α-monooxygenase (EC 1.14.17.3), herein referred to as PAM, and peptidylamidoglycolate lyase (EC 4.3.2.5), herein referred to as PGL. The preparation and purification of such PAM enzymes is familiar to the skilled worker and has been described in detail (M. Nogudi et al. Prot. Expr. Purif. 2003, 28: 293). An alternative to the "in vitro" amidation by means of PAM emerges when the enzyme is coexpressed in the same host cell with the precursor protein to be amidated (i.e SEQ ID NO:3). This is achieved by introducing a gene sequence which codes for a PAM activity into the host cell under the control of a host-specific regulatory sequence. This expression sequence can either be incorporated stably into the respective chromosomal DNA sequence, or be present on a second plasmid parallel to the expression plasmid for the target protein (i.e. SEQ ID NO:3), or be integrated as second expression cassette on the same vector, or be cloned in a polycistronic expression approach in phase with the gene sequence which encodes the target protein (i.e. SEQ ID NO:3) under the control of the same promoter sequence. A further method for amidation is based on the use of protein-specific self-cleavage mechanisms (Cottingham et al. Nature Biotech. Vol. 19, 974-977, 2001). The amidation processes described above start from a C terminus of the target peptide which is extended by at least one amino acid glycine or alternatively interim peptide. Alternative methods, are also described in WO2007036299. Accordingly, in some embodiments, the nucleic acid sequence encoding for the orexin polypeptide (i.e. SEQ ID NO:3) is chosen to allow the amidation of said orexin polypeptide and thus may comprise additional codons that will code for a glycine-extended precursor. Typically, the glycine-extended precursor resembles YGXX, where Y represents the amino acid that shall be amidated and X represents any amino acid so that the amidation enzyme (e.g. PAM) catalyzes the production of the amidated polypeptide from said glycine-extended precursor. In some embodiments, the glycine-extended precursor is MGRR.

In some embodiments, the polypeptide of the invention is an immunoadhesin.

As used herein, the term "immunoadhesin" designates antibody-like molecules which combine the binding specificity of a heterologous protein (an "adhesin" which is able to bind to OX1R) with the effector functions of immunoglobulin constant domains. Structurally, the immunoadhesins comprise a fusion of an amino acid sequence with the desired binding specificity to OX1R (i.e., is "heterologous"), and an immunoglobulin constant domain sequence. The adhesin part of an immunoadhesin molecule typically is a contiguous amino acid sequence comprising at least the binding site for OX1R. In some embodiments, the adhesin comprises the polypeptides characterized by SEQ ID NO:2 or SEQ ID NO:3. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

The immunoglobulin sequence typically, but not necessarily, is an immunoglobulin constant domain (Fc region). Immunoadhesins can possess many of the valuable chemical and biological properties of human antibodies. Since immunoadhesins can be constructed from a human protein sequence with a desired specificity linked to an appropriate human immunoglobulin hinge and constant domain (Fc) sequence, the binding specificity of interest can be achieved using entirely human components. Such immunoadhesins are minimally immunogenic to the patient, and are safe for chronic or repeated use.

In some embodiments, the Fc region is a native sequence Fc region. In some embodiments, the Fc region is a variant Fc region. In still another embodiment, the Fc region is a functional Fc region. As used herein, the term "Fc region" is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The adhesion portion and the immunoglobulin sequence portion of the immunoadhesin may be linked by a minimal linker. The immunoglobulin sequence typically, but not necessarily, is an immunoglobulin constant domain. The immunoglobulin moiety in the chimeras of the present invention may be obtained from IgG1, IgG2, IgG3 or IgG4 subtypes, IgA, IgE, IgD or IgM, but typically IgG1 or IgG3.

The polypeptides of the invention, fragments thereof and fusion proteins (e.g. immunoadhesin) according to the invention can exhibit post-translational modifications, including, but not limited to glycosylations, (e.g., N-linked or O-linked glycosylations), myristylations, palmitylations, acetylations and phosphorylations (e.g., serine/threonine or tyrosine).

In specific embodiments, it is contemplated that polypeptides used in the therapeutic methods of the present invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution. In example adding dipeptides can improve the penetration of a circulating agent in the eye through the blood retinal barrier by using endogenous transporters.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 60 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes. Such linkers may be used in modifying the protein or fragment of the protein described herein for therapeutic delivery.

In some embodiments, the OX1R agonist is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods.

As used herein the term "hepatocellular carcinoma" has its general meaning in the art and refers to the cancer developed in hepatocytes. In general, liver cancer indicates hepatocellular carcinoma in large. HCC may be caused by an infectious agent such as hepatitis B virus (HBV, hereinafter may be referred to as HBV) or hepatitis C virus (HCV, hereinafter may be referred to as HCV). In some embodiments, HCC results from alcoholic steatohepatitis or non-alcoholic steatohepatitis (hereinafter may be abbreviated to as "NASH"). The term also includes digestive hepatic micro-metastasis.

In some embodiments, the HCC is early stage HCC, non-metastatic HCC, primary HCC, advanced HCC, locally advanced HCC, metastatic HCC, HCC in remission, or recurrent HCC. In some embodiments, the HCC is localized resectable (i.e., tumors that are confined to a portion of the liver that allows for complete surgical removal), localized unresectable (i.e., the localized tumors may be unresectable because crucial blood vessel structures are involved or because the liver is impaired), or unresectable (i.e., the tumors involve all lobes of the liver and/or has spread to involve other organs (e.g., lung, lymph nodes, bone). In some embodiments, the HCC is, according to TNM classifications, a stage I tumor (single tumor without vascular invasion), a stage II tumor (single tumor with vascular invasion, or multiple tumors, none greater than 5 cm), a stage III tumor (multiple tumors, any greater than 5 cm, or tumors involving major branch of portal or hepatic veins), a stage IV tumor (tumors with direct invasion of adjacent organs other than the gallbladder, or perforation of visceral peritoneum), N1 tumor (regional lymph node metastasis), or M1 tumor (distant metastasis). In some embodiments, the HCC is, according to AJCC (American Joint Commission on Cancer) staging criteria, stage T1, T2, T3, or T4 HCC.

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, delay or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. Also encompassed by "treatment" is a reduction of pathological consequence of HCC. The methods of the invention contemplate any one or more of these aspects of treatment.

In some embodiments, the OX1R agonist of the invention is administered to the subject with a therapeutically effective amount.

By a "therapeutically effective amount" is meant a sufficient amount of OX1R to treat hepatocellular carcinoma at a reasonable benefit/risk ratio applicable to any medical treatment. It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. In particular, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, in particular from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

A further object of the invention relates to methods of decreasing the size of an established hepatocellular carcinoma in a patient in need thereof. The methods comprise a step of administering to the patient a therapeutically effective amount of an OX1R agonist, the amount being sufficient to decrease the size of the tumor. The methods may also include a step of identifying a patient suffering from or harboring a hepatocellular carcinoma, e.g. an established, detectable tumor. An "established" tumor is a tumor with a size sufficient to be detected using usual detection methods, e.g. usually the tumor is of a size of at least about 0.5 cm or greater in at least one dimension and is detectable e.g. by palpation, by imaging (e.g. X-ray, positron emission tomography-computed tomography (PET/CT), magnetic resonance imaging (MRI), ultrasound, etc.), or by some other means. 1-, 2- and/or 3-dimensional measurements may be used to determine tumor size and/or volume). Administration of at least one OX1R agonist results in a decrease in tumor size of, e.g. at least about 10%, and usually about 20, 30, 40, 50, 60, 70, 80, 90 or 100%, compared to the size of an equivalent (e.g. control) untreated tumor. A 100% decrease indicates complete eradication of detectable tumor.

A further object of the invention relates to methods of preventing or slowing hepatocellular carcinoma growth in a patient in need thereof, comprising administering to said patient a therapeutically effective amount of an OX1R agonist, the amount being sufficient to prevent or slow the growth of at least one hepatocellular carcinoma. The rate of tumor growth (increase in size) is slowed, for example, at least by about 10%, and usually by about 20, 30, 40, 50, 60, 70, 80, 90 or 100%, compared to the rate of growth of a comparable (e.g. control) untreated tumor. A 100% decrease in tumor growth means that the tumor stops growing (tumor growth in halted), and the tumor may even decrease in size (negative growth rate) in response to administration of the agonist.

The OX1R agonist of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

In particular, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The OX1R agonist of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifuCASK agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The OX1R agonist of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations ; time release capsules ; and any other form currently used.

In some embodiments, the OX1R agonist of the invention is used in combination with a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; and pharmaceutically acceptable salts, acids or derivatives of any of the above.

A further object of the invention relates to a method for treating a hepatocellular carcinoma in a subject in thereof comprising the steps consisting of i) determining the expression level of OX1R in a tumour tissue sample obtained from the subject, ii) comparing the expression level determined at step i) with a reference value and iii) administering the subject with a therapeutically effective amount of an OX1R agonist when the level determined at step i) is higher than the reference value.

The expression level of OX1R may be determined by any well known method in the art. For example methods for determining the quantity of mRNA are well known in the art. Typically the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Alternatively an immunohistochemistry (IHC) method may be used. IHC specifically provides a method of detecting targets in a sample or tissue specimen in situ. The overall cellular integrity of the sample is maintained in IHC, thus allowing detection of both the presence and location of the targets of interest (i.e. OX1R). Typically a sample is fixed with formalin, embedded in paraffin and cut into sections for staining and subsequent inspection by light microscopy. Current methods of IHC use either direct labeling or secondary antibody-based or hapten-based labeling. Examples of known IHC systems include, for example, EnVision(TM) (DakoCytomation), Powervision(R) (Immunovision, Springdale, AZ), the NBA(TM) kit (Zymed Laboratories Inc., South San Francisco, CA), HistoFine(R) (Nichirei Corp, Tokyo, Japan). In particular embodiment, a tumor tissue section may be mounted on a slide or other support after incubation with antibodies directed against OX1R. Then, microscopic inspections in the sample mounted on a suitable solid support may be performed. For the production of photomicrographs, sections comprising samples may be mounted on a glass slide or other planar support, to highlight by selective staining the presence of the proteins of interest.

A "reference value" can be a "threshold value" or a "cut-off value". Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. Typically, the threshold value is derived from the OX1R expression level (or ratio, or score) determined in a tumor tissue sample derived from one or more subjects having sufficient amount of OX1R level to get an efficient treatment with the OX1R agonist. Furthermore, retrospective measurement of the OX1R expression levels (or ratio, or scores) in properly banked historical subject samples may be used in establishing these threshold values.

A further object of the invention relates to a method for screening a drug for the treatment of hepatocellular carcinoma comprising the steps of i) providing a plurality of test substances ii) determining whether the test substances are OX1R agonists and iii) positively selecting the test substances that are OX1R agonists.

Typically, the screening method of the invention involves providing appropriate cells which express the orexin-1 receptor on their surface. Such cells include cells from mammals, yeast, Drosophila or E. coli. In particular, a polynucleotide encoding the orexin-1 receptor is used to transfect cells to express the receptor. The expressed receptor is then contacted with a test substance and an orexin-1 receptor ligand (e.g. orexins), as appropriate, to observe activation of a functional response such as recruitment of SHP-2 and induction of cell apoptosis of the cell. Functional assays may be performed as described in El Firar A, Voisin T, Rouyer-Fessard C, Ostuni MA, Couvineau A, Laburthe M. Discovery of a functional immunoreceptor tyrosine-based switch motif in a 7-transmembrane-spanning receptor: role in the orexin receptor OX1R-driven apoptosis. FASEB J. 2009 Dec; 23(12):4069-80. doi: 10.1096/fj.09-131367. Epub 2009 Aug 6. In particular comparison steps may involve to compare the activity induced by the test substance and the activity induce by a well known OX1R agonist such as orexin. In particular substances capable of having an activity similar or even better than a well known OX1R agonist are positively selected.

Typically, the screening method of the invention may also involve screening for test substances capable of binding of to orexin-1 receptor present at cell surface. Typically the test substance is labelled (e.g. with a radioactive label) and the binding is compared to a well known OX1R agonist such as orexin. The preparation is incubated with labelled OX1R and complexes of test substances bound to NGAL are isolated and characterized according to routine methods known in the art. Alternatively, the OX1R may be bound to a solid support so that binding molecules solubilized from cells are bound to the column and then eluted and characterized according to routine methods. In another embodiment, a cellular compartment may be prepared from a cell that expresses a molecule that binds NGAL such as a molecule of a signalling or regulatory pathway modulated by NGAL. The preparation is incubated with labelled NGAL in the absence or the presence of a candidate compound. The ability of the candidate compound to bind the binding molecule is reflected in decreased binding of the labelled ligand.

Typically, the candidate compound is selected from the group consisting of small organic molecules, peptides, polypeptides or oligonucleotides.

The test substances that have been positively selected may be subjected to further selection steps in view of further assaying its properties for the treatment of hepatocellular carcinoma. For example, the candidate compounds that have been positively selected may be subjected to further selection steps in view of further assaying its properties on animal models for hepatocellular carcinoma.

The above assays may be performed using high throughput screening techniques for identifying test substances for developing drugs that may be useful to the treatment of hepatocellular carcinoma. High throughput screening techniques may be carried out using multi-well plates (e.g., 96-, 389-, or 1536-well plates), in order to carry out multiple assays using an automated robotic system. Thus, large libraries of test substances may be assayed in a highly efficient manner. More particularly, stably-transfected cells growing in wells of micro-titer plates (96 well or 384 well) can be adapted to high through-put screening of libraries of compounds. Compounds in the library will be applied one at a time in an automated fashion to the wells of the microtitre dishes containing the transgenic cells described above. Once the test substances which activate the apoptotic signals are identified, they can be positively selected for further characterization. These assays offer several advantages. The exposure of the test substance to a whole cell allows for the evaluation of its activity in the natural context in which the test substance may act. Because this assay can readily be performed in a microtitre plate format, the assays described can be performed by an automated robotic system, allowing for testing of large numbers of test samples within a reasonably short time frame. The assays of the invention can be used as a screen to assess the activity of a previously untested compound or extract, in which case a single concentration is tested and compared to controls. These assays can also be used to assess the relative potency of a compound by testing a range of concentrations, in a range of 100 µM to 1 nM, for example, and computing the concentration at which the apoptosis is maximal.

The present invention also relates to a method for imaging hepatocellular carcinoma and/or digestive hepatic micro-metastasis in a subject in need thereof comprising the steps of i) administering the subject with an amount of OX1R ligand labelled with a detectable substance and ii) detecting the presence of the ligand in the subject.

As used herein the term "OX1R ligand" has its general meaning in the art and refers to any molecule (natural or not) which has the ability to bind to OX1R. Typically, OXR ligands include but are not limited to polypeptides that are functional equivalent of Orexin-A or Orexin-B, and OX1R antibodies.

As used herein, the term "labeled", with regard to OX1R ligand, is intended to encompass direct labeling of OX1R ligand by coupling (i.e., physically linking) a detectable substance.

Typically detectable substances include but are not limited to radio-imaging agents, optical imaging agents, PET imaging agents, MRI contrast agents, CT contrast agents, and FRET imaging agents.

The detectable substance may be a radioactive metal ion, i.e. a radiometal. Suitable radiometals can be either positron emitters such as 64Cu, 48V, 52Fe, 55Co, 94mTc or 68Ga; gamma-emitters such as 99mTc 111In 113mIn or 67Ga or beta-emitters such as 67Cu, 89Sr, 90Y, 153Sm, 136Re, 188Re or 192Ir. In this particular embodiment polyamino polycarboxylate chelators such as diethylenetriaminepentaacetic acid (DTPA) or 1,4,7,10-tetraazacyclododecane-N,N',N",N"'-tetraacetic acid (DOTA) were also developed for labeling radiometals. These chelators allow labeling with radioisotopes suitable for imaging such as 111In for single photon emission computed tomography (SPECT) and 68Ga for positron emission tomography (PET). Polyamino polycarboxylates are also suitable chelators for 90Y and 177Lu isotopes.

The detectable substance may be a paramagnetic metal ion, suitable such metal ions include: Gd(III), Mn(II), Cu(II), Cr(III), Fe(III), Co(II)1 Er(II)1 Ni(II), Eu(III) or Dy(III).

The detectable substance may be a gamma-emitting radioactive halogen. The radiohalogen is suitably chosen from 1231, 1311 or 77Br.

The detectable substance may be a positron-emitting radioactive non-metal. Suitable such positron emitters include: 11C, 23N, 150, 17F, 18F, 75Br, 76Br or 1241.

The detectable substance may be a hyperpolarised NMR-active nucleus. Such NMR-active nuclei may have a non-zero nuclear spin, and include 13C, 15N, 19F, 29Si and 31P. By the term "hyperpolarised" is meant enhancement of the degree of polarisation of the NMR-active nucleus over its' equilibrium polarisation. The natural abundance of 13C (relative to 12C) is about 1%, and suitable 13C-labelled compounds are suitably enriched to an abundance of at least 5%, preferably at least 50%, most preferably at least 90% before being hyperpolarised. At least one carbon atom of the imaging agent of the invention is suitably enriched with 13C, which is subsequently hyperpolarised.

The detectable substance may be a reporter suitable for in vivo optical imaging, the reporter is any moiety capable of detection either directly or indirectly in an optical imaging procedure. The reporter might be a light scatterer (e.g. a coloured or uncoloured particle), a light absorber or a light emitter. More preferably the reporter is a dye such as a chromophore or a fluorescent compound. The dye can be any dye that interacts with light in the electromagnetic spectrum with wavelengths from the ultraviolet light to the near infrared. Most preferably the reporter has fluorescent properties. Typical organic chromophoric and fluorophoric reporters include groups having an extensive delocalized electron system, e.g. cyanines, merocyanines, indocyanines, phthalocyanines, naphthalocyanines, triphenylmethines, porphyrins, pyrilium dyes, thiapyrilium dyes, squarylium dyes, croconium dyes, azulenium dyes, indoanilines, benzophenoxazinium dyes, benzothiaphenothiazinium dyes, anthraquinones, napthoquinones, indathrenes, phthaloylacridones, trisphenoquinones, azo dyes, intramolecular and intermolecular charge-transfer dyes and dye complexes, tropones, tetrazines, b/s(dithiolene) complexes, bistbenzene-dithiolatei complexes, iodoaniline dyes, b/s(S,O-dithiolene) complexes. Fluorescent proteins, such as green fluorescent protein (GFP) and modifications of GFP that have different absorption/emission properties are also useful. Complexes of certain rare earth metals (e.g., europium, samarium, terbium or dysprosium) are used in certain contexts, as are fluorescent nanocrystals (quantum dots).

Particular examples of chromophores which may be used include: fluorescein, sulforhodamine 101 (Texas Red), rhodamine B, rhodamine 6G, rhodamine 19, indocyanine green, Cy2, Cy3, Cy3.5, Cy5, Cy5.5, Cy7, Marina Blue, Pacific Blue, Oregon Green 88, Oregon Green 514, tetramethylrhodamine, and Alexa Fluor 350, Alexa Fluor 430, Alexa Fluor 532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, and Alexa Fluor 750.

Particularly preferred are dyes which have absorption maxima in the visible or near infrared (NIR) region, between 400 nm and 3 µm, particularly between 600 and 1300 nm. Optical imaging modalities and measurement techniques include, but not limited to: luminescence imaging; endoscopy; fluorescence endoscopy; optical coherence tomography, transmittance imaging; time resolved transmittance imaging; confocal imaging; nonlinear microscopy; photoacoustic imaging; acousto-optical imaging; spectroscopy; reflectance spectroscopy; interferometry; coherence interferometry; diffuse optical tomography and fluorescence mediated diffuse optical tomography (continuous wave, time domain and frequency domain systems), and measurement of light scattering, absorption, polarisation, luminescence, fluorescence lifetime, quantum yield, and quenching.

Preferred detectable substances are those which can be detected externally in a non-invasive manner following administration in vivo. Most preferred imaging moieties are radioactive, especially radioactive metal ions, gamma-emitting radioactive halogens and positron- emitting radioactive non-metals, particularly those suitable for imaging using Single photon emission computed tomography (SPECT) or Positron Emission Tomography (PET).

Techniques for conjugating detectable molecule to polypeptides and in particular antibodies, are well-known in the art (See, e.g., Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Deiker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. See also, e.g., PCT publication WO 89/12624.) Typically, the nucleic acid molecule is covalently attached to lysines or cysteines on the antibody, through N-hydroxysuccinimide ester or maleimide functionality respectively. Methods of conjugation using engineered cysteines or incorporation of unnatural amino acids have been reported to improve the homogeneity of the conjugate (Axup, J.Y., Bajjuri, K.M., Ritland, M., Hutchins, B.M., Kim, C.H., Kazane, S.A., Halder, R., Forsyth, J.S., Santidrian, A.F., Stafin, K., et al. (2012). Synthesis of site-specific antibody-drug conjugates using unnatural amino acids. Proc. Natl. Acad. Sci. USA 109, 16101-16106.; Junutula, J.R., Flagella, K.M., Graham, R.A., Parsons, K.L., Ha, E., Raab, H., Bhakta, S., Nguyen, T., Dugger, D.L., Li, G., et al. (2010). Engineered thio-trastuzumab-DMl conjugate with an improved therapeutic index to target humanepidermal growth factor receptor 2-positive breast cancer. Clin. Cancer Res.16, 4769-4778.). Junutula et al. (2008) developed cysteine-based site-specific conjugation called "THIOMABs" (TDCs) that are claimed to display an improved therapeutic index as compared to conventional conjugation methods. Conjugation to unnatural amino acids that have been incorporated into the antibody is also being explored for ADCs; however, the generality of this approach is yet to be established (Axup et al., 2012). In particular the one skilled in the art can also envisage Fc-containing polypeptide engineered with an acyl donor glutamine-containing tag (e.g., Gin-containing peptide tags or Q- tags) or an endogenous glutamine that are made reactive by polypeptide engineering (e.g., via amino acid deletion, insertion, substitution, or mutation on the polypeptide). Then a transglutaminase, can covalently crosslink with an amine donor agent (e.g., a small molecule comprising or attached to a reactive amine) to form a stable and homogenous population of an engineered Fc-containing polypeptide conjugate with the amine donor agent being site- specifically conjugated to the Fc-containing polypeptide through the acyl donor glutamine- containing tag or the accessible/exposed/reactive endogenous glutamine (WO 2012059882).

The amount of the labelled ligand used in the method of imaging described herein will depend on a variety of factors, including the identity of the targeting ligand, the identity of the detectable substance, available means for detecting the imaging agent, the means used to administer the ligand to the subject, and the weight of the subject. Generally, the ligands will be administered to a subject in a method of imaging in an amount of between about 0.1 mg to about 50 mg. Particular examples include about 0.5 mg to about 10 mg, and from about 1 mg to about 5 mg. In non-limiting examples, about 1 mg, 2 mg, 3 mg, 4 mg, 5 mg, 6 mg, 7 mg, 8 mg, 9 mg or 10 mg of a ligand of the invention may be a suitable amount of ligand to be administered to a subject for use in the methods of imaging described herein.

Typically the ligand is administered to the subject parenterally, e.g., intradermally, subcutaneously, intramuscularly, intraperitoneally, intravenously, or intrathecally. However, the skilled artisan will understand that in some instances, administration may be oral, sublingual, intranasal, intraocular, rectal, transdermal, mucosal, pulmonary, or topical.

In addition to detecting the ligands, the skilled artisan will understand that the amount of signal detected can be compared to other values, such as control values from a subject known to not have hepatocellular carcinoma and/or digestive hepatic micro-metastasies, or values obtained on an earlier date from the same subject. In some embodiments, the method further comprises measuring the amount of the ligand in the subject, and optionally further comparing the measured amount to a control value or a value obtained at an earlier time in the same subject.

Suitable means for detecting a ligand of the invention comprising a radio-imaging agent include, but are not limited to radioimaging systems, MRI, SPECT-CT, and PET imaging. Suitable means for detecting a ligand comprising an optical imaging agent include, but are not limited to flow cytometry, confocal microscopy, fluorescence activated cell sorters, and fluorescence imaging systems such as Lumina II.

Once the hepatocellular carcinoma and/or digestive hepatic micro-metastasis are detected, the subject may be treated with a OX1R agonist according to the invention.

The invention will be further illustrated by the following figures and examples. However, these examples and figures should not be interpreted in any way as limiting the scope of the present invention.

### FIGURES:

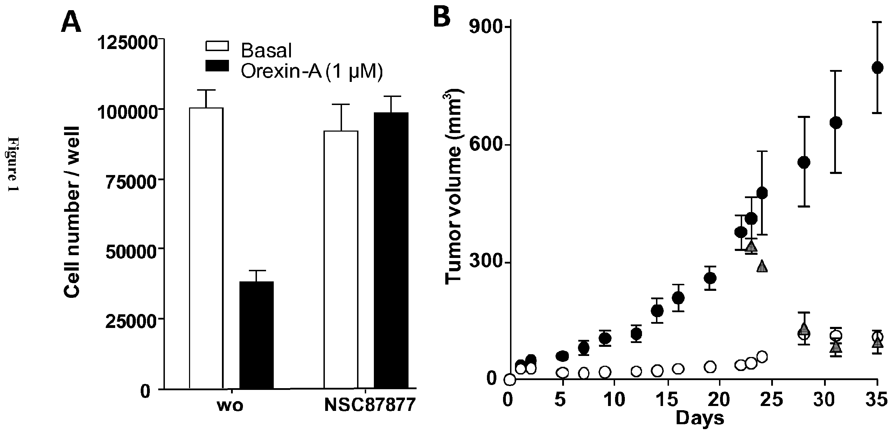

**Figure 1****.** Panel A : SHP protein tyrosine phosphatase inhibitor NSC-87877 blocks orexin-induced SK-HEP-1 growth inhibitory. SK-HEP-1 cells were challenged with (black) or without (white) 1 µM orexin-A for 48 h in the absence (wo) or presence of NSC-87877 (50 µM). At the end of the treatment, adherent cells were trypsinized, and cells excluding trypan blue were counted to measure SK-HEP-1 cell growth. Panel B : Effect of orexin-A inoculation on the growth of tumors developed by xenografting human HCC cancer cells SK-HEP-1 in nude mice. SK-HEP-1 cells were inoculated in the flank of nude mice at day 0. Mice were 2 times a week injected intraperitoneally with 100 µL of orexin-A (0,23 µmol/kg) solutions starting at day 0 (white circle) or day 22 (triangle) or with 100 mL of PBS (black circle) for controls.

### EXAMPLE:

Orexins are hypothalamic peptides involved in sleep/wake control. We have shown that orexins promote robust apoptosis in colorectal cancer cells (Voisin T, El Firar A, Fasseu M, Rouyer-Fessard C, Descatoire V, Walker F, Paradis V, Bedossa P, Henin D, Lehy T, Laburthe M. (2011) Aberrant expression of OX1 receptors for orexins in colon cancers and liver metastases: an openable gate to apoptosis. Cancer Res. 71:3341-51.). The cell death is mediated by the orexin 1 receptor (OX1R) through an original mechanism involving the presence of two ITIMs (immunoreceptor tyrosine inhibitory motif) in the OX1R sequence and the recruitment and activation of the tyrosine phosphatase SHP-2 (Voisin T, El Firar A, Rouyer-Fessard C, Gratio V, Laburthe M. (2008) A hallmark of immunoreceptor, the tyrosine-based inhibitory motif ITIM, is present in the G protein-coupled receptor OX1R for orexins and drives apoptosis: a novel mechanism. FASEB J. 22:1993-2002.). OX1R, a class A GPCR, is aberrantly expressed in primary colorectal tumors, pancreatic adenocarcinoma and liver metastases. HCC is a primary malignancy of the liver and represents the third leading cause of cancer deaths worldwide. The mainstay of treatment is resection and chemotherapy but many tumours are unresectable at presentation. We have demonstrated the expression of OX1R by immunohistochemistry in HCC using Tissue Microarray (TMA) suggesting the ectopic OX1R expression.The aims of this study were: 1/ to investigate the presence of OX1R in human HCC cell lines and to analyze orexin-A effects in relation to apoptosis; 2/ to develop an in vivo heterotopic xenograft model from the cell lines expressing OX1R, for the study of tumor growth in response to Orexin-A. The expression of OX1R was studied at mRNA (RT-PCR), proteins (immunocytochemistry) and functional levels in 4 HCC cell lines (Hep G2, SK-HEP-1, HuH-7 and Hep 3B). Only SK-HEP-1 cell line expresses OX1R. The treatment with Orexin-A promoted a 62% cell growth inhibition by promoting a mitochondrial apoptosis (Figure 1, panel A). Using the SHP inhibitor NSC-87877, we demonstrated the ability of the inhibitor to reverse orexin-induced apoptosis in SK-HEP-1 cells (Figure 1, panel A). Orexin-A injection in nude mice xenografted with SK-HEP-1 cells, has declined 80% of tumor progression in treated cases (Figure 1, panel B). Induction of apoptosis was observed in Orexin-A treated tumors (activated caspase-3).

This work has demonstrated the antitumor and proapoptotic effects of orexins in HCC. In this context, orexin receptors represent a new promising target in hepatocellular carcinoma antineoplastic therapy and/or preclinical diagnostic.

### REFERENCES:

Throughout this application, various references describe the state of the art to which this invention pertains.

## Claims

1. An OX1R agonist for use in the treatment of hepatocellular carcinoma in a subject in need thereof.

2. The OX1R agonist for use according to claim 1 wherein the OX1R agonist is an antibody, such as chimeric antibody, humanized antibody or full human monoclonal antibody.

3. The OX1R agonist for use according to claim 1 wherein the OX1R agonist is a polypeptide.

4. The OX1R agonist for use according to claim 1 wherein the OX1R agonist is a polypeptide having at least 80% of identity with SEQ ID NO:2 or 3.

5. The OX1R agonist for use according to claim 1 wherein the OX1R agonist is an immunoadhesin.

6. The OX1R agonist for use according to claim 1 wherein the OX1R is an aptamer.

7. The OX1R agonist for use according to claim 1 wherein the hepatocellular carcinoma is selected from the group consisting of early stage hepatocellular carcinoma, non-metastatic hepatocellular carcinoma, primary hepatocellular carcinoma, advanced hepatocellular carcinoma, locally advanced hepatocellular carcinoma, metastatic hepatocellular carcinoma, hepatocellular carcinoma in remission, or recurrent hepatocellular carcinoma.

8. The OX1R agonist for use according to claim 1 wherein the hepatocellular carcinoma is localized resectable, localized unresectable, or unresectable.

9. The OX1R agonist for use according to claim 1 wherein the subject is also administered with a chemotherapeutic agent.

10. An OX1R agonist for use in a method for treating a hepatocellular carcinoma in a subject in need thereof, wherein said method comprises the steps consisting of i) determining the expression level of OX1R in a tumour tissue sample obtained from the subject, ii) comparing the expression level determined at step i) with a reference value and iii) administering the subject with a therapeutically effective amount of theOXIR agonist when the level determined at step i) is higher than the reference value.

11. An OX1R agonist for use in a method for imaging hepatocellular carcinoma and/or digestive hepatic micro-metastasis in a subject in need thereof, wherein said method comprising the steps of i) administering the subject with an amount of the OX1R ligand labelled with a detectable substance and ii) detecting the presence of the OX1R ligand labelled with a detectable substance in the subject.

12. An OX1R agonist for use in a method of decreasing the size of an established hepatocellular carcinoma in a patient in need thereof, said method comprises a step of administering to the patient a therapeutically effective amount of the OX1R agonist, the amount being sufficient to decrease the size of the tumor.

13. An OX1R agonist for use in a method of preventing or slowing hepatocellular carcinoma growth in a patient in need thereof, said method comprises administering to said patient a therapeutically effective amount of the OX1R agonist, the amount being sufficient to prevent or slow the growth of at least one hepatocellular carcinoma.

## Patentansprüche

1. OX1R-Agonist zur Verwendung in der Behandlung von hepatozellulären Karzinomen bei einem Subjekt, das diese benötigt.

2. OX1R-Agonist zur Verwendung nach Anspruch 1, wobei der OX1R-Agonist ein Antikörper, wie ein chimärischer Antikörper, humanisierter Antikörper oder vollhumaner monoklonaler Antikörper ist.

3. OX1R-Agonist zur Verwendung nach Anspruch 1, wobei der OX1R-Agonist ein Polypeptid ist.

4. OX1R-Agonist zur Verwendung nach Anspruch 1, wobei der OX1R-Agonist ein Polypeptid ist, das mindestens 80 % Identität mit SEQ-ID Nr. 2 oder 3 aufweist.

5. OX1R-Agonist zur Verwendung nach Anspruch 1, wobei der OX1R-Agonist ein Immunoadhäsin ist.

6. OX1R-Agonist zur Verwendung nach Anspruch 1, wobei der OX1R ein Aptamer ist.

7. OX1R-Agonist zur Verwendung nach Anspruch 1, wobei das hepatozelluläre Karzinom ausgewählt ist aus einer Gruppe, bestehend aus hepatozellulärem Karzinom im Frühstadium, nichtmetastatischem hepatozellulären Karzinom, primärem hepatozellulären Karzinom, fortgeschrittenem hepatozellulären Karzinom, lokal fortgeschrittenem hepatozellulären Karzinom, metastatischem hepatozellulären Karzinom, hepatozellulärem Karzinom in Remission oder rekurrentem hepatozellulären Karzinom.

8. OX1R-Agonist zur Verwendung nach Anspruch 1, wobei das hepatozelluläre Karzinom lokal resezierbar ist, lokal unresezierbar ist oder unresezierbar ist.

9. OX1R-Agonist zur Verwendung nach Anspruch 1, wobei dem Subjekt auch ein chemotherapeutisches Mittel verabreicht wird.

10. OX1R-Agonist zur Verwendung in einem Verfahren zum Behandeln eines hepatozellulären Karzinoms in einem Subjekt, das dies benötigt, wobei das Verfahren umfasst die Schritte i) Bestimmen des Expressionsniveaus von OX1R in einer Tumorgewebeprobe, die vom Subjekt entnommen wurde, ii) Vergleichen des in Schritt i) bestimmten Expressionsniveau mit einem Bezugswert und iii) Verabreichen einer therapeutisch wirksamen Menge des OX1R-Agonisten an das Subjekt, wenn das in Schritt i) bestimmte Niveau höher ist als der Bezugswert.

11. OX1R-Agonist zur Verwendung in einem Verfahren zur Bildgebung von hepatozellulären Karzinomen und/oder von Mikrometastasen im Verdauungstrakt oder der Leber bei einem Subjekt, das dies benötigt, wobei das Verfahren umfasst die Schritte i) Verabreichen an das Subjekt einer Menge von OX1R-Ligand, der mit einer erkennbaren Substanz gekennzeichnet ist, und ii) Erkennen der Präsenz des mit einer erkennbaren Substanz gekennzeichneten OX1R-Liganden im Subjekt.

12. OX1R-Agonist zur Verwendung in einem Verfahren zum Verringern der Größe eines festgestellten hepatozellulären Karzinoms bei einem Patienten, der dies benötigt, wobei das Verfahren einen Schritt des Verabreichens einer therapeutisch wirksamen Menge des OX1R-Agonisten an den Patienten umfasst, wobei die Menge ausreichend ist, um die Größe des Tumors zu verringern.

13. OX1R-Agonist zur Verwendung in einem Verfahren zum Verhindern oder Verlangsamen des Wachstums eines hepatozellulären Karzinoms in einem Patienten, der dies benötigt, wobei das Verfahren das Verabreichen einer therapeutisch wirksamen Menge des OX1R-Agonisten an den Patienten umfasst, wobei die Menge ausreichend ist, um das Wachstum von zumindest einem hepatozellulären Karzinom zu verhindern oder zu verlangsamen.

## Revendications

1. Agoniste de l'OX1R pour une utilisation dans le traitement du carcinome hépatocellulaire chez un sujet en ayant besoin.

2. Agoniste de l'OX1R pour une utilisation selon la revendication 1 dans lequel l'agoniste de l'OX1R est un anticorps, tel qu'un anticorps chimérique, un anticorps humanisé ou un anticorps monoclonal totalement humain.

3. Agoniste de l'OX1R pour une utilisation selon la revendication 1 dans lequel l'agoniste de l'OX1R est un polypeptide.

4. Agoniste de l'OX1R pour une utilisation selon la revendication 1 dans lequel l'agoniste de l'OX1R est un polypeptide présentant au moins 80 % d'identité avec SEQ ID NO : 2 ou 3.

5. Agoniste de l'OX1R pour une utilisation selon la revendication 1 dans lequel l'agoniste de l'OX1R est une immunoadhésine.

6. Agoniste de l'OX1R pour une utilisation selon la revendication 1 dans lequel l'OX1R est un aptamère.

7. Agoniste de l'OX1R pour une utilisation selon la revendication 1 dans lequel le carcinome hépatocellulaire est sélectionné à partir du groupe constitué par le carcinome hépatocellulaire au stade précoce, le carcinome hépatocellulaire non métastatique, le carcinome hépatocellulaire primitif, le carcinome hépatocellulaire avancé, le carcinome hépatocellulaire avancé localement, le carcinome hépatocellulaire métastatique, le carcinome hépatocellulaire en rémission, ou le carcinome hépatocellulaire récurrent.

8. Agoniste de l'OX1R pour une utilisation selon la revendication 1 dans lequel le carcinome hépatocellulaire est localisé et résécable, localisé et non résécable, ou non résécable.

9. Agoniste de l'OX1R pour une utilisation selon la revendication 1 dans lequel le sujet reçoit également l'administration d'un agent chimiothérapeutique.

10. Agoniste de l'OX1R pour une utilisation dans un procédé de traitement d'un carcinome hépatocellulaire chez un sujet en ayant besoin, dans lequel ledit procédé comprend les étapes consistant à i) déterminer le taux d'expression de l'OX1R dans un échantillon de tissu tumoral obtenu du sujet, ii) comparer le taux d'expression déterminé à l'étape i) à une valeur de référence et iii) administrer au sujet une quantité thérapeutiquement efficace de l'agoniste de l'OX1R lorsque le taux déterminé à l'étape i) est supérieur à la valeur de référence.

11. Agoniste de l'OX1R pour une utilisation dans un procédé d'imagerie d'un carcinome hépatocellulaire et/ou de micrométastases hépatiques digestives chez un sujet en ayant besoin, dans lequel ledit procédé comprenant les étapes i) d'administration au sujet d'une quantité du ligand de l'OX1R marqué avec une substance détectable et ii) de détection de la présence du ligand de l'OX1R marqué avec une substance détectable chez le sujet.

12. Agoniste de l'OX1R pour une utilisation dans un procédé de diminution de la taille d'un carcinome hépatocellulaire établi chez un patient en ayant besoin, ledit procédé comprend une étape d'administration au patient d'une quantité thérapeutiquement efficace de l'agoniste de l'OX1R, la quantité étant suffisante pour diminuer la taille de la tumeur.

13. Agoniste de l'OX1R pour une utilisation dans un procédé de prévention ou de ralentissement de la croissance d'un carcinome hépatocellulaire chez un patient en ayant besoin, ledit procédé comprend l'administration audit patient d'une quantité thérapeutiquement efficace de l'agoniste de l'OX1R, la quantité étant suffisante pour prévenir ou ralentir la croissance d'au moins un carcinome hépatocellulaire.
